# EUROPEAN PATENT APPLICATION

(11) **EP 1 475 630 A1**
(43) Date of publication of application: **10.11.2004**
(21) Application number: 03703323.0
(22) Date of filing: 10.02.2003
(51) Int. Cl.: G01N 27/28

(54) **MEASURING DEVICE AND REMOVAL DEVICE FOR STORED OBJECT**

(30) Priority: 12.02.2002 JP 2002034211
(71) Applicant: Arkray, Inc., Kyoto-shi, Kyoto 601-8045 (JP)
(72) Inventor: Matsumoto, Daisuke, c/o Arkray, Inc., Kyoto-shi, Kyoto 601-8045 (JP)
(74) Representative: Pluckrose, Anthony William
(86) International application number: PCT/JP2003/001405
(87) International publication number: WO 2003/069326

(57) **Abstract**

A measuring device (A) includes a cartridge mount portion (6), a take-out device (B) for taking out a sensor (S) from a cartridge (2), and a measurement circuit (31) for performing measurement by utilizing the sensor (S) taken out from the cartridge (2) by the take-out device (B). The take-out device (B) includes a film breaker (70a) for breaking a film of the cartridge (2), and a movable member (70) for moving the sensor (S) to make the sensor (S) in the cartridge (2) pass through the broken portion of the film.

## Description

### TECHNICAL FIELD

The present invention relates to a measuring device used for measuring e.g. the glucose level in human blood, while also relating to a stored object take-out device to be incorporated in the measuring device for use.

### BACKGROUND ART

For a diabetic patient, it is preferable to regularly measure his or her glucose level in blood and take an appropriate measure such as medicine administration in accordance with the measurement. An example of prior art measuring device used for such a purpose is disclosed in the gazette of JP-A-8-262026.

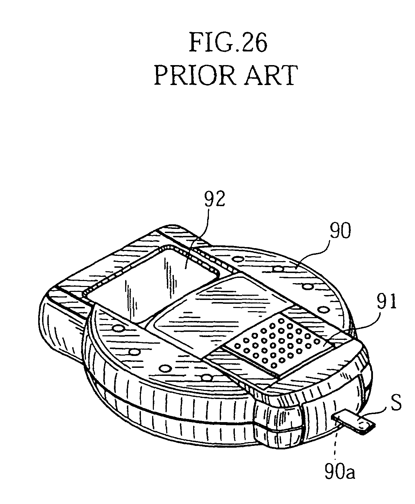

As shown in Fig. 26, the measuring device disclosed in this published document includes a housing 90 having an upper surface provided with an operation portion 91 which, when operated, causes a sensor S to partially project through an opening 90a at the front end of the housing 90. The sensor S is a small piece provided with a reagent for reacting with glucose in blood. When the blood of the user is applied to the sensor S, the measurement circuit (not shown) in the housing 90 measures the glucose level in blood, and the measurement result is displayed on a display 92.

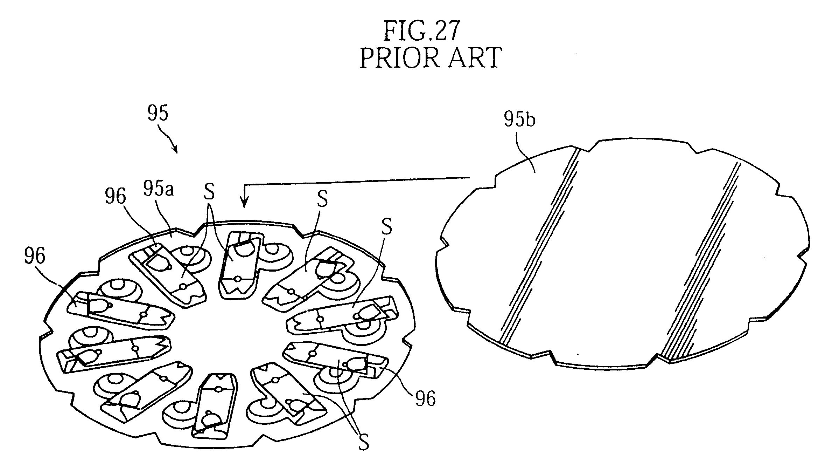

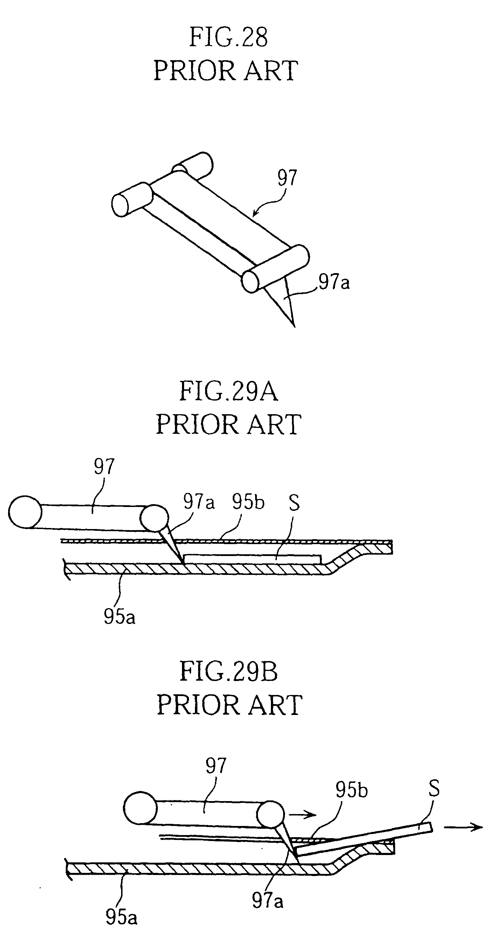

In the housing 90, a package 95 as a cartridge as shown in Fig. 27 is attached. The package 95 includes a base member 95a formed with a plurality of radially extending recesses 96 for accommodating sensors S. For protecting the sensors S, the upper surface of the base member 95a is covered with a film 95b. In the housing 90 is provided a take-out device (not shown) which operates to take out the sensors S one by one from the package 95 and cause the sensor to project outward through the opening 90a. The take-out device, the specific description of which is omitted, includes a movable member 97 provided with a blade 97a, as shown in Fig. 28. As shown in Figs. 29A and 29B, when the operation portion 91 is operated, the blade 97a of the movable member 97 breaks through part of the film 95b of the package 95 and then pushes the rear end of the sensor S toward the outer circumference of the package 95. As a result, the sensor S breaks through part of the film 95b and is pushed to the opening 90a of the housing 90.

With such a structure, by setting the package 95 at a predetermined portion, measurement of the glucose level in blood can be performed a plurality of times by successively utilizing the plurality of sensors S.

However, the prior art device has the following problems.

When the sensor S is taken out from the package 95, the sensor S itself breaks through the film 95b. Therefore, the front end of the sensor S needs to be made sharp. However, since the user may touch the front end of the sensor S, the sharp front end may cause the user to fear and hence is not preferable. Moreover, the sensor S, even with a sharp front end, may not break the film 95b easily, so that taking out of the sensor S by breaking the film 95b by the sensor S itself is sometimes difficult.

### DISCLOSURE OF THE INVENTION

An object of the present invention is to provide a take-out device and a measuring device which are capable of eliminating or lessening the above problems.

According to a first aspect of the present invention, there is provided a stored object take-out device for taking out an object from a package in which the object is stored and sealed with a wrapping member. The take-out device comprises a breaker for breaking the wrapping member, and a movable member for moving the stored object to make the stored object pass through a broken portion of the wrapping member.

Preferably, the movable member is reciprocally movable toward and away from the wrapping member and capable of holding the stored object upon contacting or approaching the stored object in the package, and the breaker comprises a blade provided at a front end of the movable member and capable of breaking through the wrapping member when the movable member moves toward the wrapping member.

Preferably, the movable member and the blade are integrally made of a metal plate.

Preferably, the movable member is provided with a holding piece in the form of a hook and capable of being flexibly deformed in moving within the package and elastically restoring to an original configuration when moved beyond the object, and the restoration causes the holding piece to move to hook the object.

Preferably, the breaker comprises a cutting blade capable of forming an opening elongated in a predetermined direction in the wrapping member, and the movable member enters the package through the opening and then pushes the object out of the package through the opening.

According to a second aspect of the present invention, there is provided a measuring device comprising a cartridge mount portion for mounting a cartridge which is a package in which a plurality of objects are stored and sealed with a wrapping member, a take-out device for taking out each of the stored objects from the cartridge when the cartridge is mounted to the cartridge mount portion, and a measurement circuit for performing measurement by utilizing the stored object taken out by the take-out device. The take-out device includes a breaker for breaking the wrapping member, and a movable member for moving the stored object to make the stored object pass through a broken portion of the wrapping member.

Preferably, the movable member is reciprocally movable toward and away from the wrapping member and capable of holding the stored object upon contacting or approaching the stored object in the package, and the breaker comprises a blade provided at a front end of the movable member and capable of breaking through the wrapping member when the movable member moves toward the wrapping member.

Preferably, the measuring device further comprises a housing accommodating the take-out device and the measuring circuit, and the housing is formed with an opening for exposing each of the stored objects. The take-out device is capable of performing a first operation for keeping the stored object exposed through the opening after the stored object is taken out from the cartridge, and a second operation for taking out the stored object to an outside of the housing through the opening.

Preferably, the measuring device further comprises a terminal electrically connected to the measurement circuit and brought into contact with the stored object during the first operation, and the contact between the terminal and the stored object enables predetermined measurement operation utilizing the measurement circuit.

Preferably, the measuring device further comprises an operation member for operating the take-out device, and the take-out device performs the second operation when the operation member is operated again after the first operation is performed by operating the operation member.

Preferably, the movable member of the take-out device is capable of holding the stored object after the stored object is taken out from the cartridge, and the take-out device is provided with a slider which advances in a predetermined direction by the operation of the operation member to push the stored object held by the movable member toward the opening of the housing to perform the first operation and the second operation.

Preferably, the take-out device is provided with a base member for guiding the slider, and the base member is formed with a cam groove for controlling movement of the slider so that the slider advances closer to the opening of the housing in the second operation than in the first operation.

Preferably, the take-out device is capable of taking out the objects stored in the cartridge through a predetermined discharge portion provided in the cartridge mount portion, and the take-out device includes a cartridge operation mechanism for operating the cartridge in cooperation with the take-out device to successively transfer the stored objects to the discharge portion every time the take-out device takes out one of the stored objects.

Preferably, the cartridge operation mechanism includes a rotor having an outer circumferential surface formed with a plurality of first projections at an upper portion thereof and a lower circumferential edge formed with a plurality of second projections, a cylindrical portion accommodating the rotor and having a circumferential wall and a bottom wall respectively formed with a plurality of third projections and fourth projections, and a resilient member applying a resilient force to the rotor to bias the rotor upward. Each of the first through the fourth projections has a tapered surface, and every time the rotor is pushed down against the resilient force, sliding movement occurs between the tapered surface of the first projection and the tapered surface of the third projection and between the tapered surface of the second projection and the tapered surface of the fourth projection to turn the rotor by a constant pitch, the cartridge turning in accordance with the turn of the rotor.

Preferably, the take-out device includes a slider capable of advancing and retreating in a predetermined direction, the slider in advancing causes the movable member to extract the stored object from the cartridge, and the rotor is pushed down every time the slider advances.

Preferably, the cartridge mount portion has a structure which allows mounting of a plurality of cartridges at a time and individual detachment of the cartridges.

Preferably, the cartridge mount portion has a structure which allows mounting of a pair of cartridges each having a circumference like a semicircular arc and combined into the form of a ring.

Preferably, the cartridge mount portion has a structure which allows mounting of a cartridge which accommodates stored objects which are sensors each provided with a reagent.

Other features and advantages of the present invention will become clearer from the description given below of the embodiments of the present invention.

### BRIEF DESCRIPTION OF THE DRAWINGS

Fig. 1 is a perspective view showing the appearance of a measuring device according to an embodiment of the present invention.
Fig. 2 is a schematic sectional view showing the measuring device of Fig. 1.
Fig. 3A shows an example of cartridge accommodating sensors, whereas Fig. 3B is an exploded perspective view of the cartridge.
Fig. 4 is a perspective view showing a take-out device incorporated in the measuring device of Fig. 1.
Fig. 5 is an exploded perspective view showing the take-out device of Fig. 4.
Fig. 6 is a perspective view showing a slider provided in the take-out device of Fig. 4.
Fig. 7 is a perspective view showing a principal portion of a movable member provided in the take-out device of Fig. 4.
Figs. 8A-8D each is a sectional view of a principal portion for showing the operation for taking out a sensor.
Fig. 9 is a sectional view taken along lines IX-IX in Fig. 4.
Fig. 10 is a sectional view showing a principal portion of Fig. 9.
Fig. 11 is an exploded perspective view showing, partially in section, a principal portion of Fig. 9.
Figs. 12A-12D illustrate the operation of the portion shown in Fig. 11.
Fig. 13 is a side view, partially in section, showing the take-out device of Fig. 4.
Fig. 14 is a side view, partially in section, showing the operation of the take-out device of Fig. 4.
Fig. 15 is a side view, partially in section, showing the operation of the take-out device of Fig. 4.
Fig. 16 is a sectional view showing the operation of the take-out device of Fig. 4.
Figs. 17A and 17B each is a schematic plan view of a principal portion for showing the operation of the take-out device of Fig. 4.
Fig. 18 is a sectional view showing the operation of the take-out device of Fig. 4.
Fig. 19 is a plan view showing a principal portion of the take-out device of Fig. 4.
Figs. 20A-20C schematically illustrate another embodiment of the present invention.
Fig. 21 is a perspective view showing another example of cartridge.
Fig. 22 is a sectional view showing a principal portion of another example of cartridge.
Fig. 23 is a sectional view showing a principal portion of another example of cartridge.
Fig. 24 is a perspective view showing another example of cartridge.
Fig. 25 schematically illustrates another embodiment of the present invention.
Fig. 26 is a perspective view showing the appearance of a prior art measuring device.
Fig. 27 is a perspective view showing the prior art cartridge and film covering sensors.
Fig. 28 illustrates the operation of the prior art device.
Figs. 29A and 29B illustrate the operation of the prior art device.

### BEST MODE FOR CARRYING OUT THE INVENTION

Preferred embodiments of the present invention will be described below in detail with reference to the accompanying drawings.

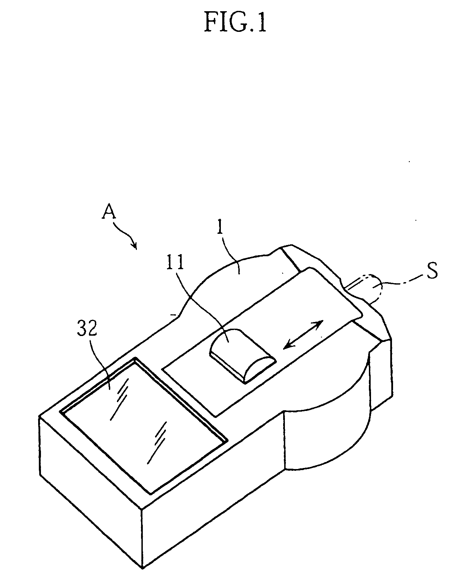

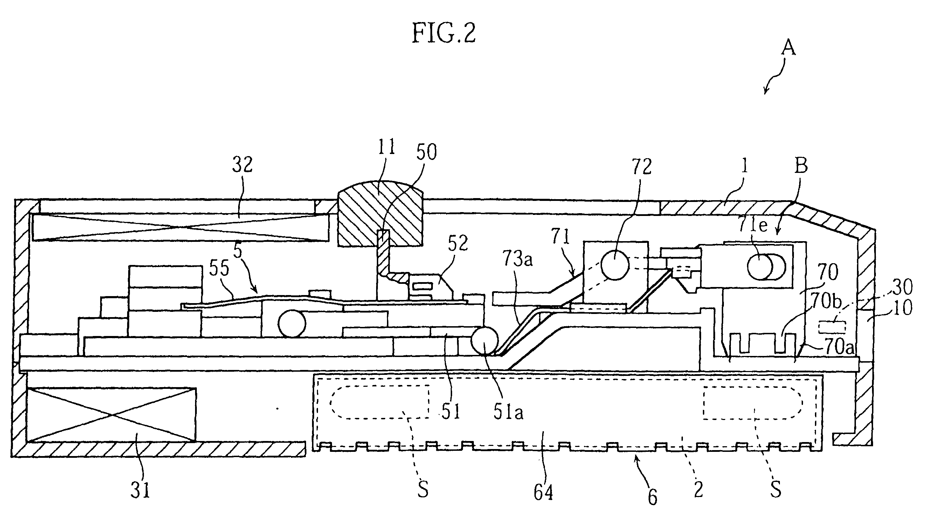

Figs. 1 and 2 show an example of measuring device according to the present invention. As shown in these figures, the measuring device A in this embodiment includes a housing 1, and a take-out device B accommodated in the housing 1.

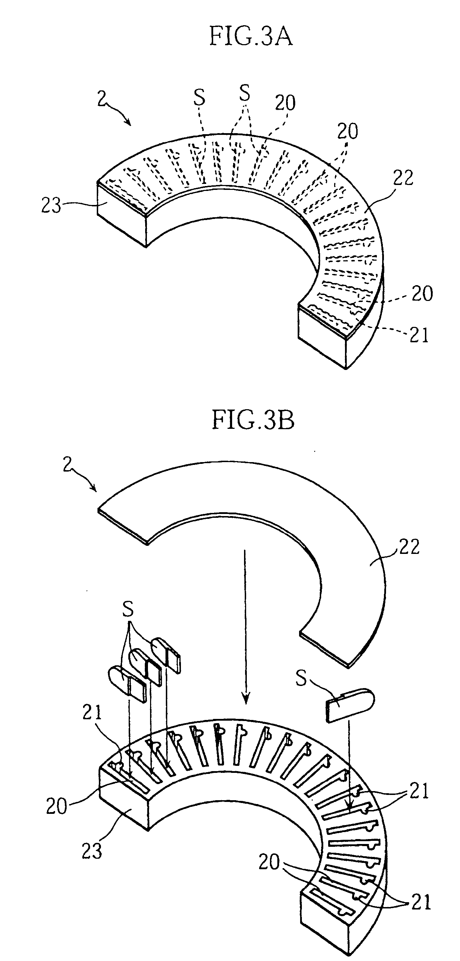

The measuring device A is capable of measuring the glucose level in blood by utilizing a plurality of sensors S stored in a cartridge 2 shown Figs. 3A and 3B. Each of the sensors S is in the form of a small piece provided with a reagent for reacting with glucose and an electrode for contacting the reagent, which are not illustrated in the figure.

The cartridge 2 includes a case 23 in the form of a generally half ring and made of a synthetic resin. The case 23 is formed with a plurality of upwardly open grooves 20 extending radially and equally spaced. The sensors S are accommodated in the grooves 20 while standing widthwise. Each of the grooves 20 is provided with a small semicircular recess 21 communicating with the groove. The recess 21 accommodates a desiccant (not shown) to prevent the quality deterioration of the sensor due to moisture. A film 22 made of e.g. an aluminum foil is attached to the upper surface of the case 23, whereby the sensors S are tightly sealed. In use, the cartridge 2 is mounted to a cartridge mount portion 6, which will be described later.

As clearly shown in Fig. 2, the housing 1 has a side surface formed with an opening 10. The opening 10 is utilized for causing the sensor S taken out from the cartridge 2 by the take-out device B to partially project to the outside. The housing 1 incorporates a terminal 30, a measurement circuit 31, and a display 32. The terminal 30 is so arranged as to come into contact with an electrode of the sensor S when the sensor S partially projects outward through the opening 10. The measurement circuit 31 is provided with a CPU and its associated memory, for example. When human blood is introduced to the reagent of the sensor S with the terminal 30 contacting the electrode of the sensor S, the measurement circuit computes the glucose level in the blood based on the change of the current flowing through the reagent. The method for computing the glucose level is conventionally known, and the description thereof is omitted. The display 32, which may comprise a liquid crystal panel, is utilized for displaying the glucose level computed by the measurement circuit 31 or other information. The display screen of the display 32 can be seen from the outside of the housing 1.

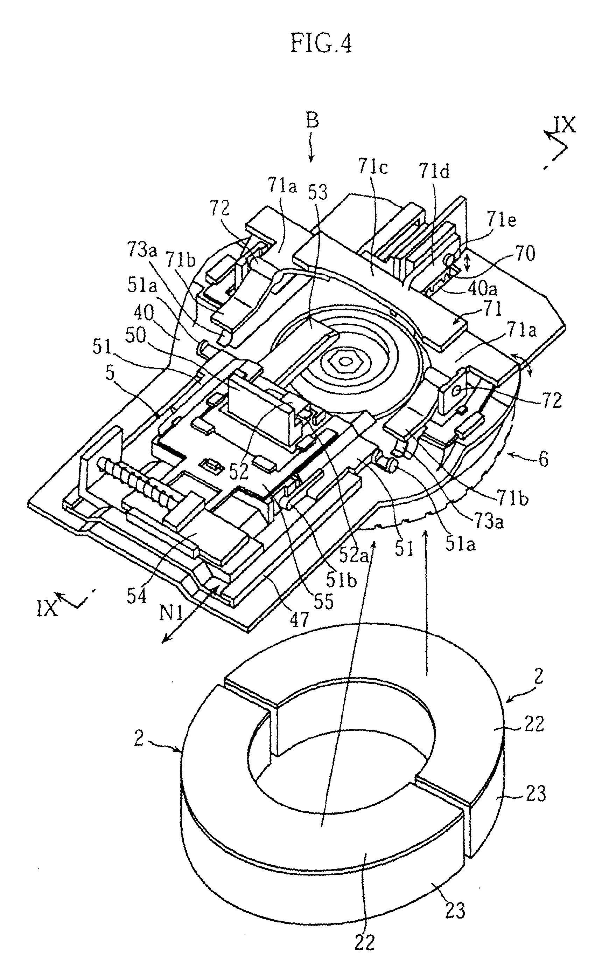

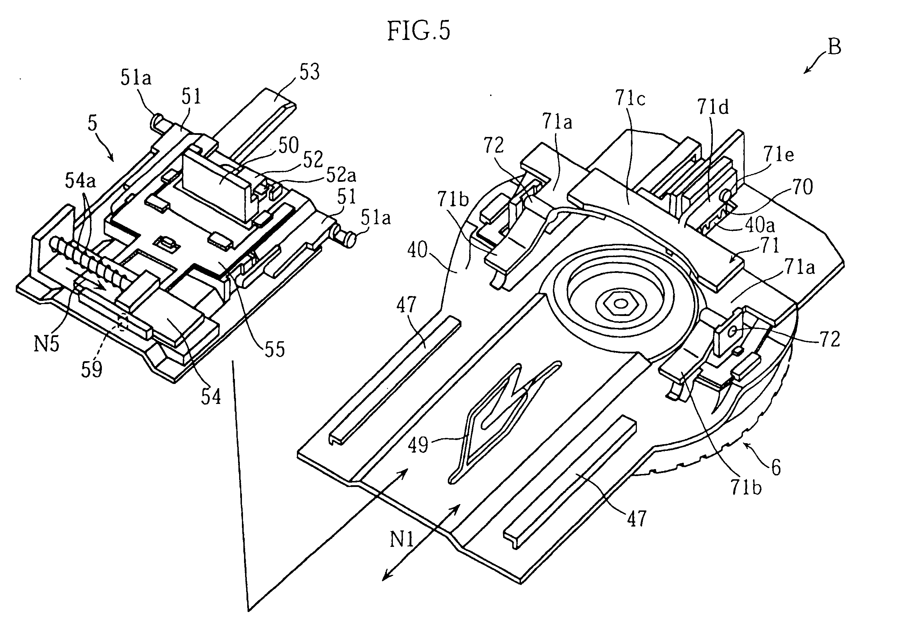

As shown in Figs. 4 and 5, the take-out device B includes a base member 40 and a slider 5 mounted on the base member 40. The base member 40 is provided, as attachment, with a cartridge mount portion 6 and a pivot arm 71 supporting a movable member 70.

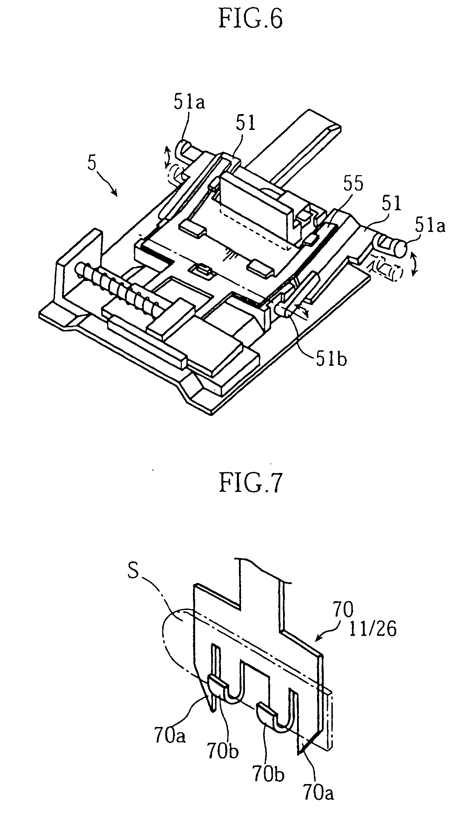

The movable member 70 serves to break through the film 22 of the cartridge 2 to hold the sensor 2 stored in the cartridge 2 and is made of hard metal into a relatively small wall thickness, for example. As shown in Fig. 7, the movable member 70 is provided with a pair of holding pieces 70b each having a lower end in the form of a hook. The movable member 70 has a lower portion integrally formed with a pair of blades 70a each having a sharp front end. The movable member 70 is movable up and down through an opening 40a penetrating the base member 40. The cartridge 2 is arranged below the base member 40 so that the movable member 70, moving up and down, can take the sensor S out of the cartridge 2 in the following manner.

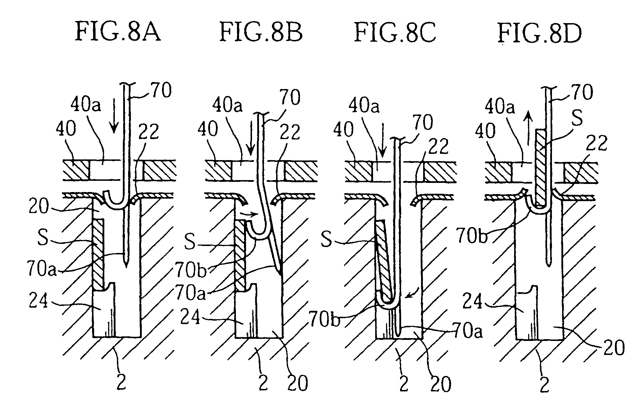

As shown in Fig. 8A, when the movable member 70 moves downward through the opening 40a of the base member 40, the paired blades 70a break through the film 22 on the upper surface of the cartridge 2. Thus, the movable member 70 enters the groove 20 of the cartridge 2. The sensor S is supported by a rib 24 and thereby suspended above the bottom surface of the groove 20. As shown in Fig. 8B, when the movable member 70 enters the groove, the front end of each holding piece 70b comes into contact with a side surface of the sensor S, whereby the hook portion of the holding piece 70b is deformed by compression. Subsequently, as shown in Fig. 8C, when the holding piece 70b descends below the sensor S, the holding piece 70b elastically restores to its original state. By this restoration, the hook portion of the holding piece 70b enters under the sensor S to come into engagement with the lower edge of the sensor S. By this engagement, the holding piece 70b holds the sensor S. Thereafter, as shown in Fig. 8D, the movable member 70 moves upward, thereby taking out the sensor S.

As shown in Figs. 4 and 5, the pivot arm 71, which can pivot upward and downward about a pair of shafts 72, is provided with paired projections 71a, each of which is connected to a corresponding one of paired projections 71b via a shaft 72 interposed therebetween. The paired projections 71a are connected to each other via a connection portion 71c. The connection portion 71c is provided with a support projection 71d projecting generally horizontally from the connection portion 71c and supporting and suspending the movable member 70 via a pin 71e. As will be described later, the pivot arm 71 pivots in accordance with the reciprocal movement of the slider 5, and the pivoting movement causes the movable member 70 to move up and down.

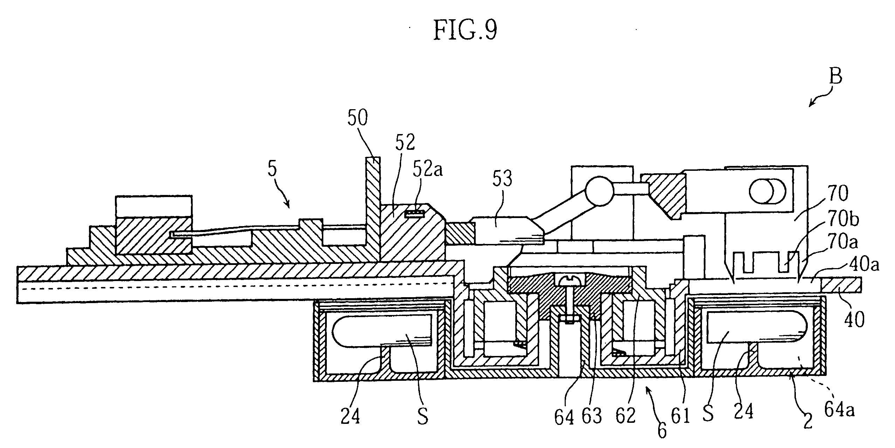

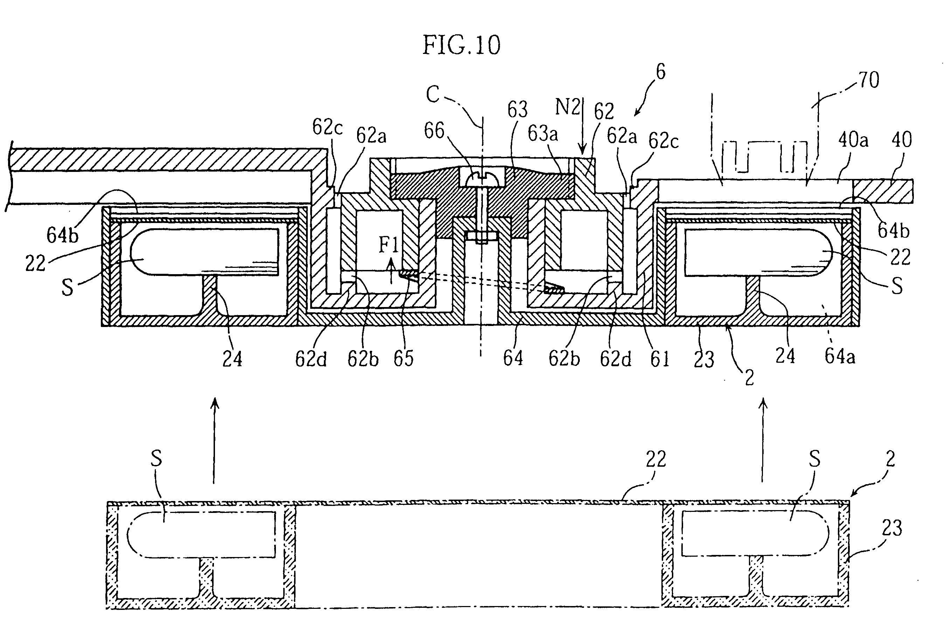

As shown in Figs. 9 and 10, the cartridge mount portion 6 includes a cylindrical portion 61, a rotor 62, a press member 63 and a holder 64.

The cylindrical portion 61 is fixed to the base member 40. The cylindrical portion 61 may be formed integrally on or separately from the base member 40. The holder 64 includes a space 64a which opens downward for removably fitting the cartridge 2. As shown in Fig. 4, two cartridges 2 are received in the holder 64 as combined together to have a ring-like shape. With this arrangement, after all of the sensors S in either one of the two cartridges 2 are used out, that cartridge can be replaced with a new one, whereby more than a predetermined number of sensors S can be constantly stocked in the holder 64. As shown in Fig. 10, the holder 64 has an upper surface formed with a plurality of openings 64b each in the form of a slit. The openings 64b serve as passages for taking out the sensors S from the cartridge 2. The cartridge 2 and the holder 64 are provided with appropriate positioning means (not shown) such as a projection or a recess for positioning each of the sensors S of the cartridge 2 directly under a respective one of the openings 64b of the holder 64 when the cartridge 2 is set in the holder 64.

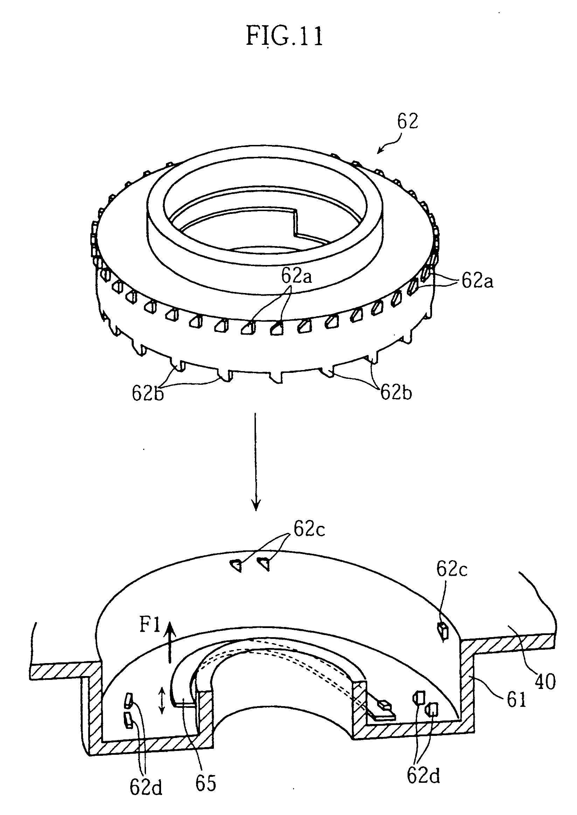

The cartridge mount portion 6 is provided with a cartridge operation mechanism for turning the holder 64 and the cartridge 2 in a predetermined direction about the central axis C of the cylindrical portion 61 at a constant pitch every time the rotor 62 is pressed in the direction indicated by the arrow N2. Specifically, as shown in Fig. 11, the cartridge operation mechanism includes a plurality of first projections 62a provided at a predetermined pitch at an upper portion of the outer circumferential surface of the rotor 62. The lower circumferential edge of the rotor 62 is formed with a plurality of second projections 62b arranged at a constant pitch. Between the bottom surface of the rotor 62 and the bottom of the cylindrical portion 61 is interposed a spring 65. The spring 65 has a resilient force F1 constantly biasing the rotor 62 upward. As shown in Fig. 10, the press member 63 is provided with a collar portion 63a capable of coming into engagement with part of the rotor 62, thereby preventing the rotor 62 from moving above a predetermined height due to the resilient force F1. The cylindrical portion 61 has a circumferential wall and a bottom wall formed with a plurality of third projections 62c and a plurality of fourth projections 62d, respectively. The third projections and the fourth projections engage the first projections 62a and the second projections 62b of the rotor 62, respectively, thereby turning the rotor 62, as follows.

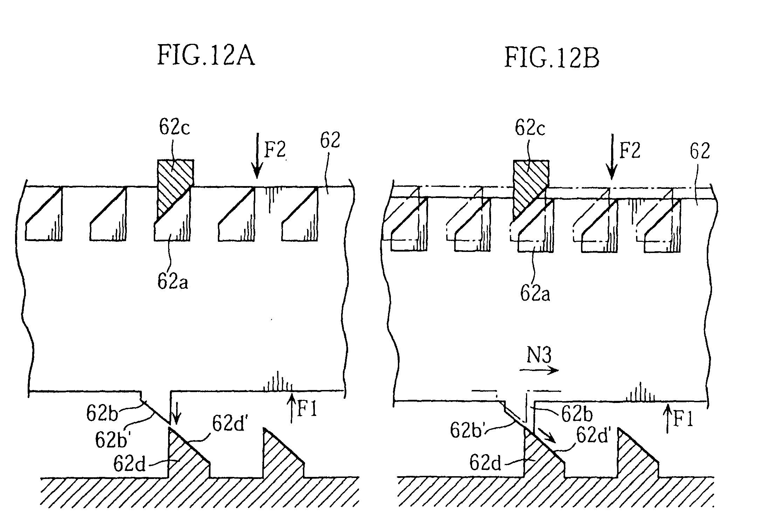

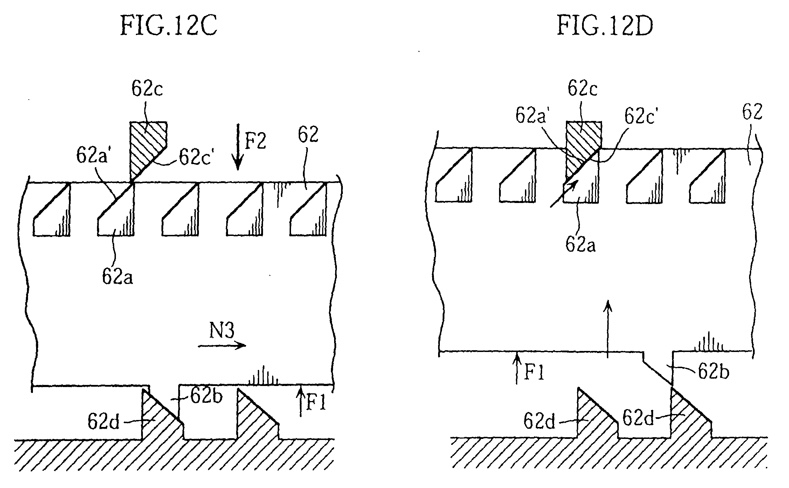

As shown in Fig. 12A, the rotor 62 is normally held at a predetermined top dead center by the resilient force F1. In this state, when the rotor 62 is pushed downward with an appropriate force F2 against the resilient force F1, a tapered surface 62b' of the second projection 62b comes into contact with a tapered surface 62d' of the fourth projection 62d, as shown in Fig. 12B. As a result, due to the sliding movement between the tapered surfaces 62b' and 62d', the rotor 62 turns in the direction indicated by the arrow N3 in the figure. As shown in Fig. 12C, when the rotor 62 descends to a predetermined bottom dead center while turning, the upper end of one of the first projections 62a is located forward of the lower end of one of the third projection 62c in the direction of rotation of the rotor 62. Therefore, when the force F2 to press the rotor 62 downward is thereafter released so that the rotor 62 moves upward due to the resilient force F1 of the spring 65, the tapered surface 62c' of the third projection 62c slides on the tapered surface 62a' of the first projection 62a. As a result, due to the sliding movement between the tapered surfaces 62a' and 62c', the rotor 62 further turns in the direction of the arrow N3. As shown in Fig. 12D, when the rotor 62 ascends to return to the top dead center, the positional relationship between the first through the fourth projections 62a-62d are the same as that in the initial state. Therefore, every time the rotor 62 is pushed down thereafter, the rotor 62 turns in the rotation direction noted above by a constant angle. The angle through which the rotor 62 turns by a single up-down movement corresponds to the pitch of the first projections 62a.

As shown in Fig. 10, the rotor 62 is held in engagement with the outer circumference of the collar portion 63a of the press member 63, and the press member 63 is connected to the holder 64 via a screw 66. Therefore, when the rotor 62 turns, the holder 64 turns together with the rotor. Specifically, the holder 64 turns to turn the openings 64b and the sensors S in the cartridge 2 through an angle corresponding to one pitch, thereby successively transferring the openings and the sensors to a position directly below the opening 40a. The pressing of the rotor 62 is performed at a predetermined timing by using a press member 52 of a slider 5, which will be described later.

As shown in Figs 4 and 5, the slider 5 is slidable on the base member 40 in the longitudinal direction N1 of the take-out device B while being guided by a pair of guide portions 47 formed on the base member 40. The slider 5 is provided with an operation projection 50, a pair of movable arms 51, the press member 52, a projection 53 and a movable block 54.

As shown in Fig. 2, the operation projection 50 is connected to an operation knob 11 provided on the upper surface of the housing 1. By sliding the operation knob 11, the slider 5 can be moved reciprocally. However, the present invention is not limited to such a structure. For instance, the operation projection 50 may be exposed to the outside of the housing 1 so that the user can directly operate the operation projection 50.

As shown in Fig. 6, each of the movable arms 51 of the slider 5 has a base end provided with a shaft portion 51b and a distal end provided with a horizontally extending projection 51a. The movable arm is pivotable about the shaft portion in such a manner that the projection 51a moves up and down. When the movable arm 51 moves upward, a spring 55 made of a thin metal plate is deformed so that the spring 55 exerts a force to push down the movable arm 51.

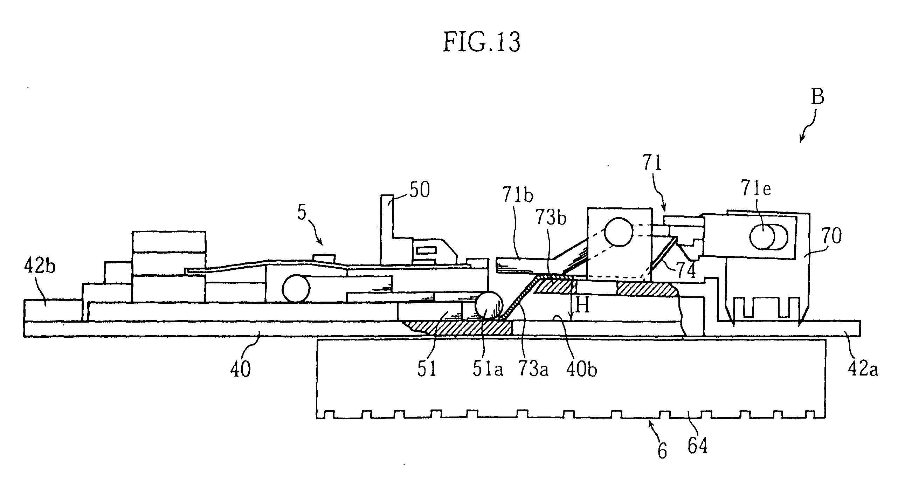

In the take-out device B, the movable member 70 moves up and down in accordance with the reciprocal sliding movement of the slider 5. Specifically, as shown in Fig. 13, in a normal state in which no operation force is exerted to the operation projection 50, the slider 5 is held at a location which is offset toward a rear end 42b of the base member 40 due to a resilient force of a spring 78, which will be described later. In this state, in front of each of the movable arms 51 (right side in the figure) is positioned a spring plate 73a which is inclined to become higher as it extends toward the front end 42a of the base member 40, and a guide plate 73b supporting the spring plate 73a. The guide plate is positioned higher, by a predetermined amount H, than the surface 40b of the base member 40 for guiding the bottom surface of the slider 5.

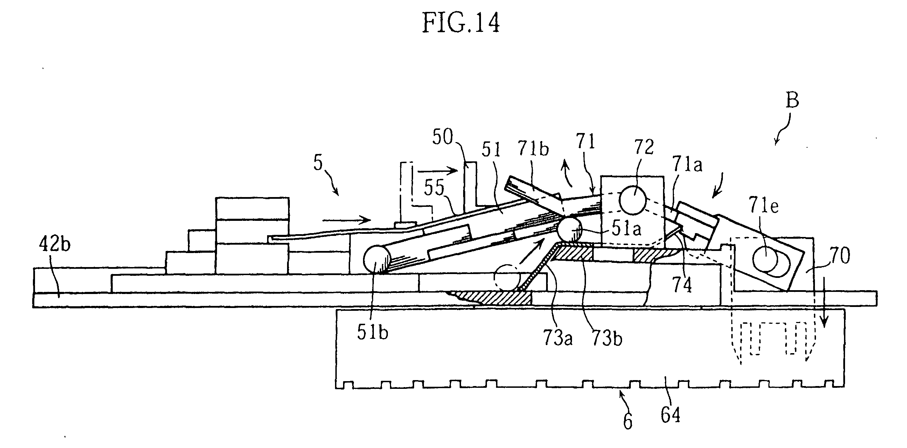

As shown in Fig. 14, when the slider 5 advances, the projection 51a of each movable arm 51 is guided by the spring plate 73a to move upward. Thus, while turning the movable arm 51 upward, the projection 51a is guided onto the guide plate 73b. Thus, the projection 51a lifts the projection 71b of the pivot arm 70, thereby turning the pivot arm 71 clockwise in the figure. As a result, the movable member 70 moves downward.

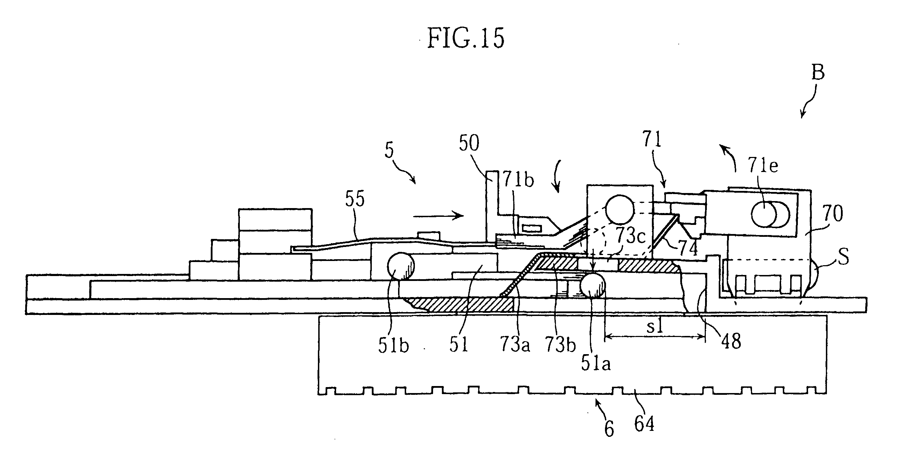

As shown in Fig. 15, when the slider 5 further advances, the projection 51a of each movable arm 51 reaches a slit 73c formed in the guide plate 73b and moves downward through the slit 73c. As a result, the projection 71b of the pivot arm 71 is released from the raised state by the projection 51a. Therefore, the pivot arm 71 turns counterclockwise due to the resilient force of the spring 74 to return to its original posture. As a result, the movable member 70 moves upward to return to its original height. After the projection 51a moves downward as noted above, the slider 5 can be further advanced by a predetermined stroke s1 until the projection 51a comes into contact with a wall 48 standing on the base member 40. Conversely, the slider 5 can be retreated to its original position shown in Fig. 13. When the slider 5 retreats, the projection 51a passes under the spring plate 73a while hitting the lower end of the spring plate 73a upward.

The press member 52 of the slider 5 serves to push down the upper portion of the rotor 62 of the cartridge mount portion 6 , and is supported by a spring plate 52a for up and down movement. The projection 53 projects forward from a front portion of the slider 5. The press member 52 and the projection 53 operate as follows.

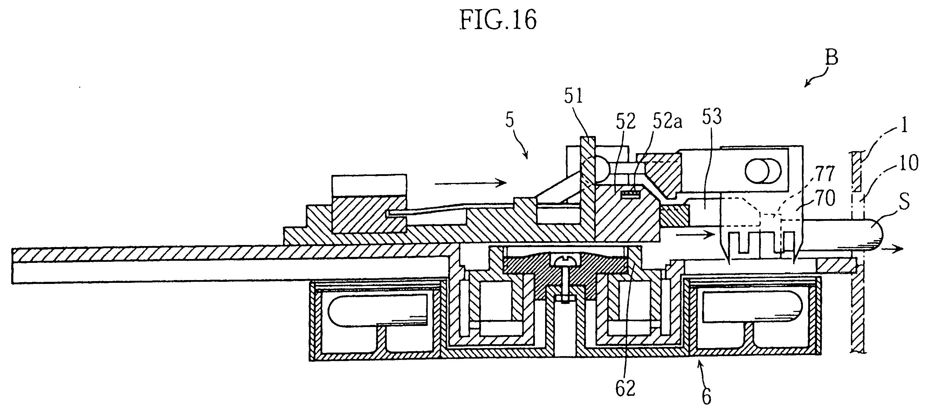

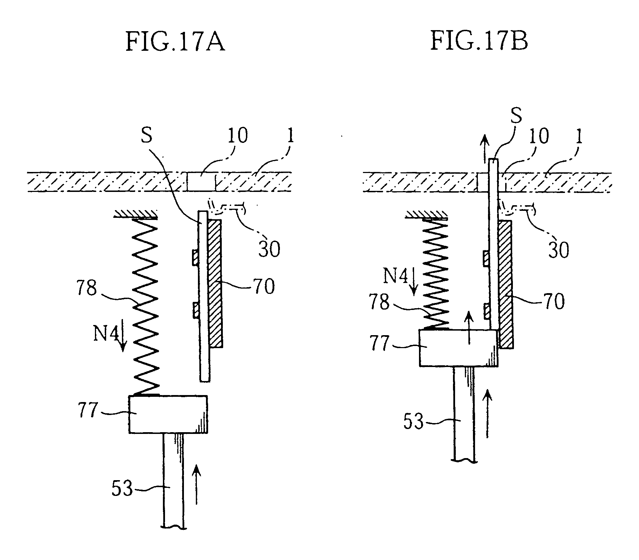

When the slider 5 is advanced as shown in Fig. 16, the press member 52 is located above the rotor 62. In this state, the press member 52 is not ready for pushing down the rotor 62. At this time, the projection 53 advances a slide block 77 by a predetermined amount. As shown in figs. 17A and 17B, the slide block 77, when pushed by the projection 53, pushes the rear end of the sensor S held by the movable member 70 to advance the sensor S. The slide block is slidably mounted on the base member 40. The slide block 77 is constantly biased in the direction indicated by the arrow N4 by the spring 78. Therefore, the advancement of the slide block 77 by the projection 53 is performed against the resilient force of the spring 78. The resilient force of the spring 78 serves to return the slider 5 to a predetermined position which is offset toward the rear end 42b of the base member 40. As shown in Fig. 17B, when the sensor S held by the movable member 70 is advanced by an appropriate amount, the electrode (not shown) of the sensor S come into contact with the terminal 30.

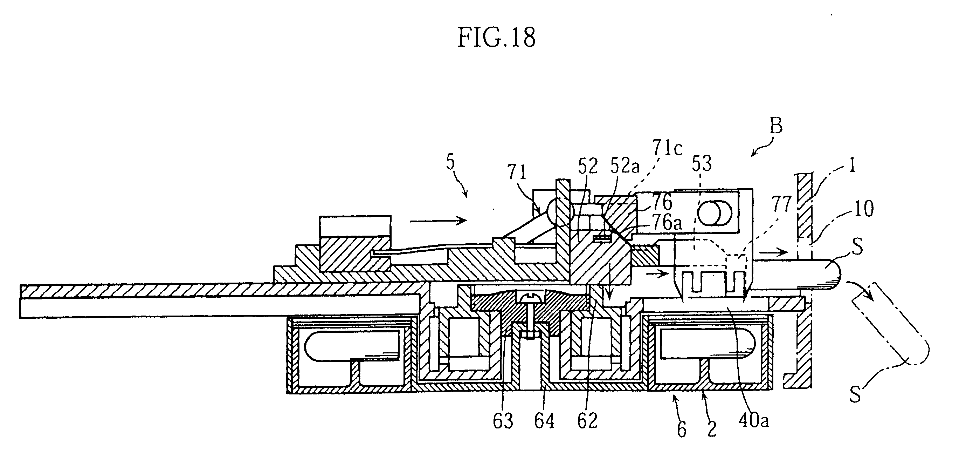

As shown in Fig. 18, when the slider 5 is further advanced, part of the press member 52 comes into engagement with an inclined surface 76a of a cam 76 provided at the connection portion 71c of the pivot arm 71. Due to the engagement, the press member 52 is guided downward as it advances, thereby pushing down the rotor 62. At this time, the projection 53 further advances the slide block 77. As a result, the slide block 77 pushes the sensor S held by the movable member 70 to disengage the sensor from the movable member 70. The sensor S pushed in this way is extracted to the outside through the opening 10 of the housing 1.

As shown in fig. 5, the base member 40 is formed with a cam groove 49, whereas the bottom surface of the movable block 54 of the slider 5 is provided with a downwardly extending pin 59 for engagement in the cam groove 49. The movable block 54 is reciprocally movable widthwise of the slider 5. The movable block 54 is constantly biased in the direction indicated by the arrow N5 due to a resilient force of a spring 54a.

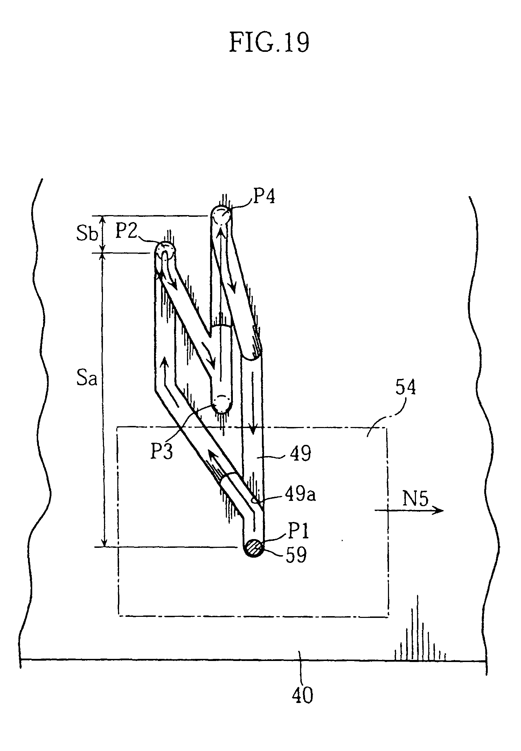

The cam groove 49 has such a configuration as shown in Fig. 19. When the slider 5 is positioned at the most retreated position, the pin 59 is located at a first position P1 in the cam groove 49. In reciprocally moving the slider 5, the pin 59 advances a predetermined distance Sa to a second position P, retreats to a thirdposition P3, advances to a fourth position P4, and then returns to the first position P1. Therefore, advancement is performed twice during the one cycle of movement of the slider 5. The fourth position P4 is located forward of the position P2 by a predetermined distance Sb. Therefore, by the second advancement, the slider 5 comes to a position forward of the position which the slider 5 reaches by the first advancement. The first and the second advancing movements of the slider 5 correspond to the advancing movements shown in Figs. 16 and 18, respectively.

When the pin 59 moves widthwise of the slider 5, the movable block 54 moves widthwise of the slider 5 so that other portions of the slider 5 do not move in that direction. As noted above, the movable block 54 is constantly biased in the direction indicated by the arrow N5. Therefore, when the pin 59 moves from the second position P2 to the third position P3 or returns from the fourth position P4 to the first position P1, the pin 59 readily moves in the direction of the arrow N5, thereby reliably moving to the intended position. When the pin 59 advances from the first position P1, the pin comes into contact with a wall 49a provided in the cam groove 49 and guided along the wall, whereby the movement of the pin directly toward the fourth position P4 is prevented.

Next, the applications and functions of the measuring device A having the above structure will be described.

First, with two cartridges 2 mounted to the cartridge mount portion 6, the operation knob 11 is advanced. As described with reference to Fig. 19, this first advancement is performed until the pin 59 reaches the second position P2. In this advancement, as shown in Figs. 14 and 15, the projection 51a of each movable arm 51 of the slider 5 causes the pivot arm 71 to pivot. As a result, the movable member 70 moves downward and then returns upward. When the movable member 70 moves downward, the blades 70a of the movable member 70 break through the film 22, and the movable member 70 holds the sensor S in the cartridge 2. By the subsequent upward movement of the movable member 70, the sensor S is taken out to a portion above the base member 40. At this time, the sensor S is taken out through a portion where the film 22 is broken with the blades 70a of the movable member 70. Therefore, unlike the prior art device, the sensor S need not break the film 22. Accordingly, the sensor S need not have a sharp edge, which provides the user with a feeling of safety.

In the first advancement of the slider 5, after the movable member 70 takes out the sensor S from the cartridge 2, the slider 5 can be advanced further, as shown in Fig. 16. When the slider 5 advances in this way, the sensor S held by the movable member 70 is pushed forward by the projection 53 and the slide block 77. As a result, part of the sensor S projects outward through the opening 10 of the housing 1. Therefore, the user can apply blood to the sensor S. As shown in Figs. 17A and 17B, the sensor S comes into contact with the terminal 30, whereby the measurement of the glucose level in blood can be performed properly by using the measurement circuit 31. When the user releases the operation knob 11, the slider 5 retreats slightly due to the resilient force of the spring 78 shown in Figs. 17A and 17B, but the sensor S is kept partially projecting through the opening 19 of the housing 1. Therefore, the continuous holding of the operation knob 11 by the user, which is a troublesome work, is not necessary.

After the process for measuring the glucose level is completed and the measurement result is displayed at the display 32, the user advances the operation knob 11 again. As shown in Fig. 18, by this second advancement, the projection 53 and the slide block 77 come forward of the position reached by the first advancement. As a result, the sensor S is taken out from the housing 1 through the opening 10. The sensor S, which is a used one, may be subjected to disposal, for example. Since the user need not hold the sensor S for disposal, it is possible to prevent the user's hand from becoming soiled with blood.

In the second advancement described above, the press member 52 comes into engagement with the cam 76, thereby moving downward. When the user releases the operation knob 11 after the disposal of the sensor S, the slider 5 returns to its initial position. Therefore, the press member 52 push down the rotor 62 of the cartridge mount portion 6 only once and then returns to its original height due to the resilient force of the spring plate 52a. When the rotor 62 is pushed down only once, the holder 64 and the cartridges 2 turn through an angle corresponding to one pitch of the sensors S in the cartridge 2 by the operation described with reference to Figs. 12A-12D. As a result, another unused sensor S is brought directly below the movable member 70 and the opening 40a. Therefore, when the operation knob 11 is operated again thereafter, the unused sensor S can be properly taken out from the cartridge 2. In this way, by successively taking out the sensors S from the cartridge 2 one by one in a predetermined order, the measurement process utilizing a sensor can be performed a plurality of times.

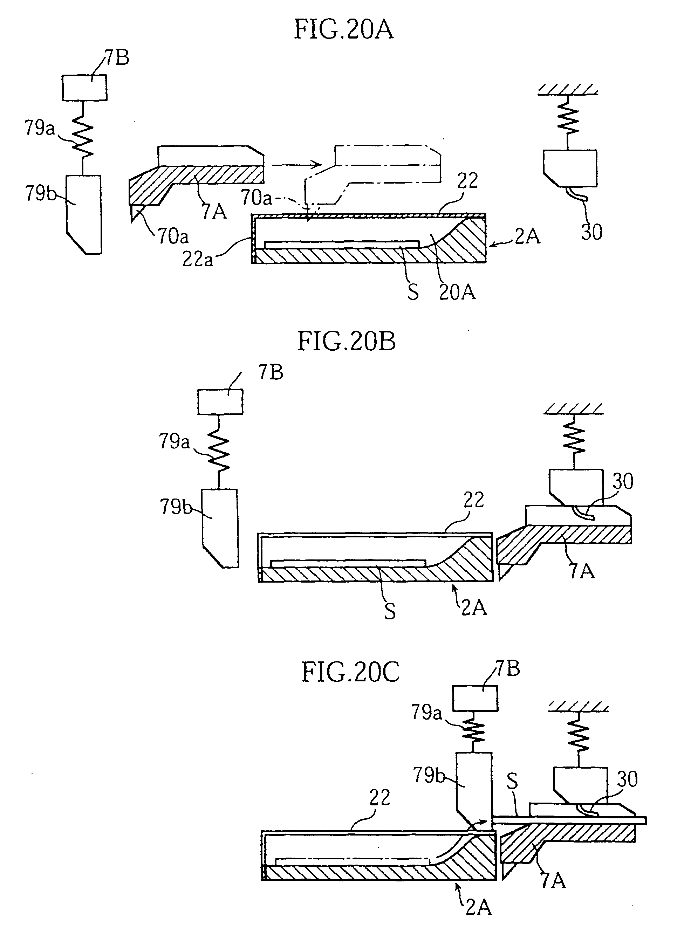

Figs. 20A-25 show other embodiments of the present invention. In these figures, the elements which are identical or similar to those of the foregoing embodiment are designated by the same reference signs as those used for the foregoing embodiment.

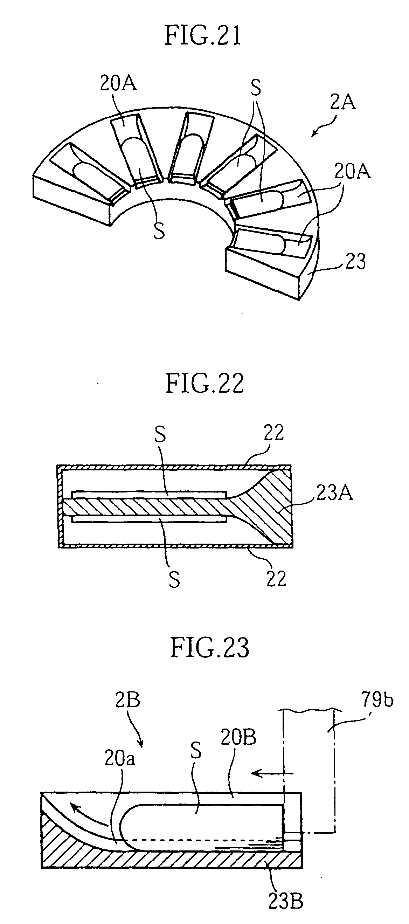

The structure shown in Fig. 20A includes, as movable members for taking out sensors S from a cartridge 2A, a first movable member 7A provided with a blade 70a and a second movable member 7B suspending a push member 79b via a spring 79a. As shown in Fig. 21, the cartridge 2A includes a case 23 having an upper surface formed with a plurality of radially extending recesses 20A. The sensors S are stored as laid in respective recesses 20A.

As indicated by phantom lines in Fig. 20A, the first movable member 7A moves horizontally so that the blade 70a continuously cuts the film 22 of the cartridge 2A. As a result, a cutout having a predetermined length is formed in the film 22. As shown in Fig. 20B, the first movable member 7A, after having passed over the cartridge 2A, waits on one side of the cartridge 2A. The second movable member 7B moves horizontally so that the push member 79b enters the cartridge 2A through the cutout of the film 22 and push the rear end of the sensor S. As shown in Fig. 20C, when the sensor S is pushed in this way, the sensor S is pushed out of the cartridge 2A through the cutout of the film 22 and guided onto the first movable member 7A. At this time, a terminal 30 for measurement comes into contact with a electrode of the sensor S.

With this structure again, the sensor S is taken out through the portion where the film 22 is cut with the blade 70a. Therefore, the sensor S need not break the film 22, and hence, need not be have a sharp edge. As shown in Fig. 20A, when the film 22 includes a portion 22a standing along the inner circumferential surface of the cartridge 2A, the portion 22a can be broken by pushing down the push member 79b or the blade 70a.

When the sensors S are stored as laid in the cartridge 2A as shown in Fig. 21, the total number of the sensors S which can be stored in the cartridge is smaller than that when the sensors are stored as standing in the cartridge. For instance, to effectively increase the number of storage of the sensors, the recesses 20A for storing the sensors S may be provided on both of the obverse and the reverse sides of the case 23A, as shown in Fig. 22. The cartridge having such a structure may also be used in the present invention.

In a cartridge 2B shown in Fig. 23, a sensor S is accommodated in a recess 20B formed in a case 23B. One edge of the sensor S is fitted in a groove 20a formed at the bottom of the recess 20B, whereby the sensor S is kept standing. With this structure again, similarly to the embodiment shown in Figs. 20A-20C, the sensor S can be taken out from the cartridge 2B by pushing the rear end of the sensor S with a push member 79b. Such means can also be used in the present invention.

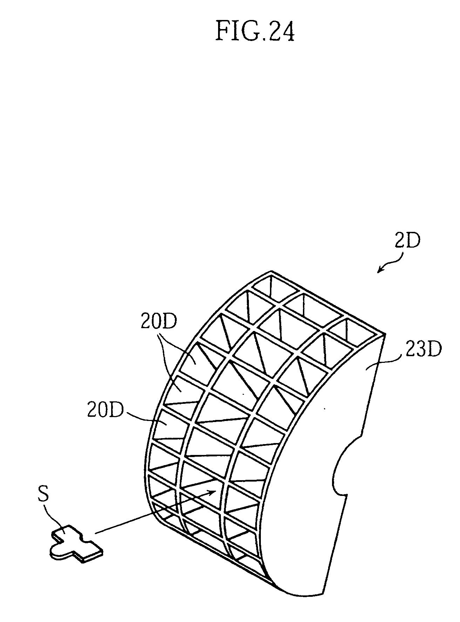

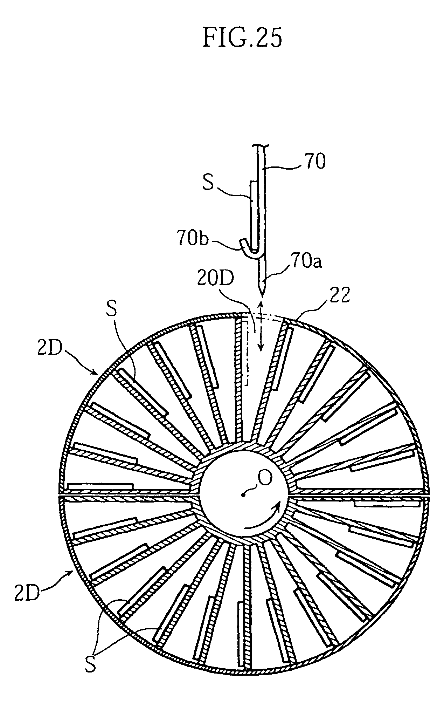

In a cartridge 2D shown in Fig. 24, a case 23D has a circumferential surface having a curvature and formed with a plurality of recesses 20D. When two cartridges 2D are combined together, a configuration like a hollow drum can be obtained, as shown in Fig. 25. Also when such cartridges 2D are used, the sensor S can be taken out in a manner similar to that in the embodiment shown in Figs. 1-19. Specifically, the blade 70a of a movable member 70 breaks through the film 22 closing each of the recesses 20D. Thereafter, the movable member 70 enters the recess 20D to hold the sensor S, and then the movable member 70 exits the cartridge 2D. By turning the two cartridges 2D about their center O, a plurality of sensors S can be transferred one by one to a position where the movable member 70 takes out the sensor S.

The present invention is not limited to the foregoing embodiments. Specific structure of each part of the stored obj ect take-out device and the measuring device may be modified in various ways.

The measuring device of the present invention is not limited to one used for the measurement of a glucose level in blood, but may be structured as a device used for various kinds of measurement in the medical field or in other technical fields. Therefore, the kind and specific structure of the sensor is not limitative. The cartridge to be mounted to the measuring device may not be provided with a hard case but may be a package containing a plurality of sensors as sealed with a soft flexible film.

The stored object take-out device of the present invention need not necessarily be incorporated in a measuring device, but may be used alone. Alternatively, the take-out device may be incorporated in a device other than a measuring device. Therefore, the kind of the package and object stored in the package is not limitative.

## Claims

1. A stored object take-out device for taking out an object from a package in which the object is stored and sealed with a wrapping member, the take-out device comprising:
a breaker for breaking the wrapping member; and
a movable member for moving the stored object to make the stored object pass through a broken portion of the wrapping member.

2. The stored object take-out device according to claim 1, wherein the movable member is reciprocally movable toward and away from the wrapping member and capable of holding the stored object upon contacting or approaching the stored object in the package; and
wherein the breaker comprises a blade provided at a front end of the movable member and capable of breaking through the wrapping member when the movable member moves toward the wrapping member.

3. The stored object take-out device according to claim 2, wherein the movable member and the blade are integrally made of a metal plate.

4. The stored object take-out device according to claim 2, wherein the movable member is provided with a holding piece in a form of a hook and capable of being flexibly deformed in moving within the package and elastically restoring to an original configuration when moved beyond the object, the restoration causing the holding piece to move to hook the object.

5. The stored object take-out device according to claim 1, wherein the breaker comprises a cutting blade capable of forming an opening elongated in a predetermined direction in the wrapping member; and
wherein the movable member enters the package through the opening and then pushes the object out of the package through the opening.

6. A measuring device comprising:
a cartridge mount portion for mounting a cartridge which is a package in which a plurality of objects are stored and sealed with a wrapping member;
a take-out device for taking out each of the stored objects from the cartridge when the cartridge is mounted to the cartridge mount portion; and
a measurement circuit for performing measurement by utilizing the stored object taken out by the take-out device;
wherein the take-out device includes a breaker for breaking the wrapping member, and a movable member for moving the stored obj ect to make the stored obj ect pass through a broken portion of the wrapping member.

7. The measuring device according to claim 6, wherein the movable member is reciprocally movable toward and away from the wrapping member and capable of holding the stored object upon contacting or approaching the stored object in the package; and
wherein the breaker comprises a blade provided at a front end of the movable member and capable of breaking through the wrapping member when the movable member moves toward the wrapping member.

8. The measuring device according to claim 6, further comprising a housing accommodating the take-out device and the measuring circuit, the housing being formed with an opening for exposing each of the stored objects;
wherein the take-out device is capable of performing a first operation for keeping the stored object exposed through the opening after the stored object is taken out from the cartridge, and a second operation for extracting the stored object to an outside of the housing through the opening.

9. The measuring device according to claim 8, further comprising a terminal electrically connected to the measurement circuit and brought into contact with the stored object during the first operation;
wherein the contact between the terminal and the stored object enables a predetermined measurement operation utilizing the measurement circuit.

10. The measuring device according to claim 8, further comprising an operation member for operating the take-out device;
wherein the take-out device performs the second operation when the operation member is operated again after the first operation is performed by operating the operation member.

11. The measuring device according to claim 10, wherein the movable member of the take-out device is capable of holding the stored object after the stored object is taken out from the cartridge; and
wherein the take-out device is provided with a slider which advances in a predetermined direction by the operation of the operation member to push the stored object held by the movable member toward the opening of the housing to perform the first operation and the second operation.

12. The measuring device according to claim 11, wherein the take-out device is provided with a base member for guiding the slider; and
wherein the base member is formed with a cam groove for controlling movement of the slider so that the slider advances closer to the opening of the housing in the second operation than in the first operation.

13. The measuring device according to claim 6, wherein the take-out device is capable of taking out the objects stored in the cartridge through a predetermined discharge portion provided in the cartridge mount portion; and
wherein the take-out device includes a cartridge operation mechanism for operating the cartridge in cooperation with the take-out device to successively transfer the stored objects to the discharge portion every time the take-out device extracts one of the stored objects.

14. The measuring device according to claim 13, wherein the cartridge operation mechanism includes a rotor having an outer circumferential surface formed with a plurality of first projections at an upper portion thereof and a lower circumferential edge formed with a plurality of second projections, a cylindrical portion accommodating the rotor and having a circumferential wall and a bottom wall respectively formed with a plurality of third projections and fourth projections, and a resilient member applying a resilient force to the rotor to bias the rotor upward;
wherein each of the first through the fourth projections has a tapered surface; and
wherein, every time the rotor is pushed down against the resilient force, sliding movement occurs between the tapered surface of the first projection and the tapered surface of the third projection and between the tapered surface of the second projection and the tapered surface of the fourth projection to turn the rotor by a constant pitch, the cartridge turning in accordance with the turn of the rotor.

15. The measuring device according to claim 14, wherein the take-out device includes a slider capable of advancing and retreating in a predetermined direction, the slider in advancing causes the movable member to extract the stored object from the cartridge; and
wherein the rotor is pushed down every time the slider advances.

16. The measuring device according to claim 6, wherein the cartridge mount portion has a structure which allows mounting of a plurality of cartridges at a time and individual detachment of the cartridges.

17. The measuring device according to claim 16, wherein the cartridge mount portion has a structure which allows mounting of a pair of cartridges each having a circumference like a semicircular arc and combined into a form of a ring.

18. The measuring device according to claim 6, wherein the cartridge mount portion has a structure which allows mounting of a cartridge which accommodates stored objects which are sensors each provided with a reagent.
